(19) Europäisches Patentamt
European Patent Office
Office européen des brevets

(11) Veröffentlichungsnummer: **0 216 324**
**B1**

(12) # EUROPÄISCHE PATENTSCHRIFT

(45) Veröffentlichungstag der Patentschrift: **22.11.90**

(51) Int. Cl.[5]: **C 07 D 207/38**

(21) Anmeldenummer: **86112951.8**

(22) Anmeldetag: **19.09.86**

(54) **4-Alkoxy-3-pyrrolin-2-on-1-yl-essigsäurealkyl- bzw. -benzylester sowie deren Herstellung.**

(30) Priorität: **24.09.85 CH 4119/85**
**14.05.86 CH 1958/85**
**19.06.86 CH 2486/85**

(43) Veröffentlichungstag der Anmeldung:
**01.04.87 Patentblatt 87/14**

(45) Bekanntmachung des Hinweises auf die Patenterteilung:
**22.11.90 Patentblatt 90/47**

(84) Benannte Vertragsstaaten:
**AT BE CH DE FR GB IT LI LU NL SE**

(56) Entgegenhaltungen:
**DE-A-2 328 277**
**DE-A-2 635 853**
**DE-A-2 635 854**
**US-A-3 299 095**

**IL FARMACO, Ed. Sc., Band 32, Heft 8, Seiten 602-613; G. PIFFERI et al.: "Cyclic gaba-gabob analogues"**

(73) Patentinhaber: **LONZA AG**
**Gampel/Wallis (CH)**

(72) Erfinder: **Meul, Thomas, Dr.**
**Balfrinstrasse 21**
**Visp (Kanton Wallis) (CH)**
Erfinder: **Tenud, Leander, Dr. Ing-Chem. ETH**
**Balfrinstrasse 23**
**Visp (Kanton Wallis) (CH)**
Erfinder: **Duc, Laurent, Dr.**
**Rue de la Meunière**
**Sion (Kanton Wallis) (CH)**
Erfinder: **McGarrity, John, Dr.**
**Rathausstrasse 5**
**Visp (Kanton Wallis) (CH)**

(74) Vertreter: **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**

**EP 0 216 324 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Beschreibung**

Die Erfinding betrifft neue 4-Alkoxy-3-pyrrolin-2-on-1-yl-essigsäurealkyl(benzyl)ester sowie ein Verfahren zu deren Herstellung.

In EP—A—224 256 sind die entsprechenden (gesättigten) Pyrrolidinverbindungen und Verfahren zu deren Herstellung beschrieben.

4-Alkoxy-3-pyrrolin-2-on-verbindungen sowie deren 1-Essigsäurealkylester sind wertvolle, stabile Zwischenprodukte für die Synthese des cerebral aktiven 4-Hydroxy-2-oxo-pyrrolidin-1-yl-acetamids.

Von Pifferi et al., Il Farmaco, Ed. Sc., *1977*, 32, 602 ist ein Verfahren bekannt, den Wirkstoff herzustellen. Eine schlechte Ausbeute und teure Ausgangsprodukte machen dieses Verfahren jedoch nicht rentabel.

Es stellte sich daher die Aufgabe, einen neuen Weg zu finden, der die genannten Nachteile ausschaltet.

Diese Aufgabe konnte auf überraschende Weise durch das Auffinden der neuen Zwischenprodukte der 4-Alkoxy-3-pyrrolin-2-on-1-yl-essigsäurealkylester der allgemeinen Formel

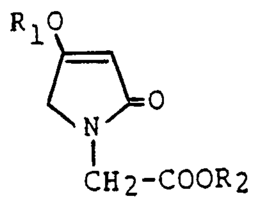

(1)

worin $R_1$ Alkyl mit 1 oder 2 C-Atomen und $R_2$ ALkyl mit 1 bis 4 C-Atomen oder Benzyl bedeutet, gelöst werden.

Eine erfindungsgemäß bevorzugte Verbindung ist der 4-Methoxy-3-pyrrolin-2-on-1-yl-essigsäureethylester.

Die neuen Verbindungen, die sich z.B. gemäß dem in der EP—A—216 325 beschriebenen Verfahren in 4-Hydroxy-2-oxo-pyrrolidin-1-yl-acetamid umwandeln lassen, sind erfindungsgemäss auf einfache Weise, gemäss einem Verfahren nach Patentanspruch 3, herstellbar.

So wird in einem ersten Schritt en 4-Halogenacetessigsäure-$C_1$—$C_4$-alkylester, vorzugsweise ein 4-Chloracetessigsäure-$C_1$—$C_4$-alkylester, besonders bevorzugt der 4-Chloracetessigsäuremethylester mit Orthoameisensäure $C_1$—$C_2$-trialkylester, bevorzugt mit dem Orthoameisensäuretrimethylester, in einem sauren Medium umgesetzt.

Als Säuren finden zweckmässig Schwefelsäure, Sulfonsäuren oder ein saurer Ionenaustauscher Anwendung.

Der resultierende 4-Halogen-3-($C_1$—$C_2$)-alkoxy-2-E-butensäure-($C_1$—$C_4$)-alkylester kann isoliert werden. Die Weiterumsetzung kann entweder direkt mit einem Glycin-$C_1$—$C_4$-alkyl- oder benzylester in Gegenwart einer Base zum Endprodukt oder indirekt zuerst mit wässriger Ammoniaklösung zum 4-($C_1$—$C_2$)-alkoxy-3-pyrrolin-2-on und weiter mit 2-Bromessigsäure-$C_1$—$C_4$-alkyl- oder benzylester in Gegenwart eines Alkalihydrids, zum Endprodukt durchgeführt werden.

Wird der direkte Weg, durch Umsetzung des Halogenalkoxybutensäureesters mit dem Glycinester gewählt, wird zweckmässig in Gegenwart von schwachen anorganischen Basen oder tert. aliphatischen Aminen als Base gearbeitet. Besonders bevorzugte Vertreter der schwachen anorganischen Basen sind z.B. Natriumacetat, Kaliumcarbonat oder Kaliumhydrogencarbonat. Als geeigneter Vertreter eines tert. aliphatischen Amins kann Triethylamin verwendet werden.

Es ist ausserdem von Vorteil, die Reaktion in einem Lösungsmittel durchzuführen. Zweckmässig werden dazu aprotische oder protische Lösungsmittel mit hoher Polarität verwendet.

Geeignete Vertreter sind z.B. Acetonitril, Propionitril, Butyronitril, Diethylenglycolmonomethylether, Diethylenglycoldimethylether, N,N'-Dimethylformamid oder N,N'Dimethylacetamid.

Zweckmässig wird der Glycin-$C_1$—$C_4$-alkyl- oder -benzylester in einer Menge von 1 bis 4 Mol, vorzugsweise von 1,25 bis 2 Mol pro Mol Halogenalkoxybutensäureester eingesetzt.

Zweckmässig kommt das Hydrogenhalogen, vorzugsweise das Hydrochlorid des Glycinalkylesters zur Anwendung.

Dann ist es notwendig, vor der Umsetzung mit dem Halogenalkoxybutensäureester durch Behandeln mit einer Base, wie z.B. einem Trialkylamin, vorzugsweise Triethylamin, oder mit einem Alkalimethylat, wie Natriummethylat, das Glycinalkylesterhydrohalogenid in den freien Glycinalkylester überzuführen.

Die Reaktionstemperatur wählt man zweckmässig zwischen 60 und 120°C.

Sofern es die Siedetemperatur des Lösungsmittels zulässt (wie z.B. bei Acetonitril), kann bei Rückflusstemperatur gearbeitet werden.

Nach beendigung der Reaktion wird auf übliche Weise, z.B. durch Extraktion und gegebenenfalls durch eine anschliessende Umkristallisation, das Produkt aufgearbeitet.

Mit diesen erfindungsgemässen Verfahren werden Ausbeuten erreicht, die in der Regel 80% übersteigen.

Die Gehalte der erhaltenen Produkte liegen üblicherweise höher als 95%.

Wird der indirekte Weg gewählt, setzt man in einem ersten Schritt den 4-Halogen-3-($C_1$—$C_2$)-alkoxy-2-E-butensäure-($C_1$—$C_4$)-alkylester, zweckmässig mit einer wassrigen $NH_3$-Lösung mit einer Konzentration von zweckmässig 10% bis 25%, vorzugsweise 15% bis 25% um. Die Reaktionstemperaturen bewegen sich zweckmässig zwischen 20 bis 100°C, vorzugsweise zwischen 60 bis 80°C.

Das Molverhältnis 4-Halogen-3-($C_1$—$C_2$)-alkoxy-2-E-butensäure—$C_1$—$C_4$-alkylester zu $NH_3$ liegt zweckmässig zwischen 1 zu 2 und 1 zu 5, vorteilhaft zwischen 1 zu 2,5 und 1 zu 3,5.

Nach üblicher Aufarbeitung, z.B. durch Extraktion mit geeigneten Lösungsmitteln aus der Gruppe der halogenierten Kohlenwasserstoffe, wie u.a. Methylenchlorid und Chloroform, kann das entsprechende 4-($C_1$—$C_2$)-alkoxy-3-pyrrolin-2-on in Ausbeuten gegen 90%, bezogen auf den 4-Halogenacetessigsäure-$C_1$—$C_4$-alkylester, erhalten werden.

Dieses kann gegebenenfalls nach zusätzlicher Reinigung, durch z.B. Umkristallisieren in aromatischen Kohlenwasserstoffen bevorzugt in Toluol, für die letzte Stufe eingesetzt werden. Darin wird das 4-($C_1$—$C_2$)-alkoxy-3-pyrrolin-2-on, bevorzugt das 4-Methoxy-3-pyrrolin-2-on, mit einem 2-Bromessigsäure-$C_1$—$C_4$-alkylester oder einem 2-Bromessigsäurebenzylester, bevorzugt mit dem 2-Bromessigsäureethylester, in Gegenwart eines Alkalihydrids, vorteilhaft mit Natriumhydrid, umgesetzt.

Das Molverhältnis der eingesetzten Verbindungen 4-($C_1$—$C_2$)-alkoxy-3-pyrrolin-2-on zu 2-Bromessigsäure-$C_1$—$C_4$-alkylester resp. 2-Bromessigsäurebenzylester zu Alkalihydrid liegt zweckmässig zwischen 1 zu 1 zu 1 und 1 zu 1,5 zu 1,4, vorzugsweise zwischen 1 zu 1,2 zu 1,2 und 1 zu 1,4 zu 1,4.

Die Reaktionstemperatur wählt man zweckmässig zwischen 0 und 50°C.

Die Reaktion führt man vorteilhaft in gegenüber Alkalihydriden inerten Lösungsmitteln, zweckmässig in Acetonitril, Tetrahydrofuran, Dimethoxyethan oder Dimethylformamid, vorzugsweise in Acetonitril oder Dimethylformamid, durch.

Es ist ebenso von Vorteil, unter einem Schutzgas wie Stickstoff oder Argon zu arbeiten.

Nach üblicher Aufarbeitung, z.B. ddurch Extraktion gegebenenfalls anschliessender Reinigung durch Kristallisation, können auch auf diesem Weg in sehr guter Ausbeute die erfindungsgemässen Zwischenprodukte erhalten werden.

Nach einer weiteren bevorzugten Ausführungsform des erfindungsgemäßen Verfahrens wird in der ersten Stufe der durch Umsetzung von 4-Halogenacetessigsäure-$C_1$—$C_4$-alkylester und Orthoameisensaure-$C_1$—$C_2$-trialkylester in saurem Medium zunächst entstehende 4-Halogen-3,3-di[($C_1$—$C_2$)-alkoxy]-butansäure-($C_1$—$C_4$)-alkylester durch Erhitzen im Vakuum in den 4-Halogen-3-($C_1$—$C_2$)-alkoxy-2-E-butensaure-($C_1$—$C_4$)-alkylester überführt und gegebenenfalls isoliert und dann wird in der zweiten Stufe gemäß Variante b) von Anspruch 3 verfahren.

Zur Erzeugung des sauren Mediums werden zweckmässig anorganische Säuren, wie Schwefelsäure oder Sulfonsäuren, wie Methansulfonsäure verwendet.

Bevorzugt gelangt Schwefelsäure zum Einsatz.

Bezogen auf 1 Mol eingesetzten 4-Halogenacetessigsäure-$C_1$—$C_4$-alkylester werden zweckmässig 0,01 bis 0,1 Mol der entsprechenden Säure verwendet.

Die Reaktionstemperatur in der ersten Stufe, der Herstellung der 4-Halogen-3,3-di-[($C_1$—$C_2$)-alkoxy]-butansäure-$C_1$—$C_4$-alkylester, bewegt sich vorteilhaft zwischen 0 und 30°C.

Die Reaktionstemperatur in der zweiten Stufe, der Herstellung der 3-($C_1$—$C_2$)-alkoxy-2-E-butensäure-($C_1$—$C_4$)-alkylester, bewegt sich vorteilhaft zwischen 100 und 160°C, bei einem Druck von 50 bis 200 mbar.

Der resultierende 3-($C_1$—$C_2$)-alkoxy-2-E-butensäure-($C_1$—$C_4$)-alkylester kann isoliert werden, vorzugsweise wird er aber direkt mit wassriger Ammoniaklösung zum Endprodukt weiterumgesetzt.

Dazu wird in der Regel so vorgegangen, dass aus dem Reaktionsgemisch der ersten Stufe durch Vakuumdestillation die flüchtigen Reaktionsprodukte entzogen werden. Durch Erhitzen im Vakuum wird in der zweiten Stufe die Abspaltung des Methanols aus dem intermediär erzeugten Ketal realisiert. Der Rückstand wird darauf mit wassriger Ammoniaklösung mit einem $NH_3$-Gehalt von zweckmässig 10 bis 25%, vorzugsweise 15 bis 25% versetzt.

Die Reaktionstemperatur bewegt zich zweckmässig zwischen 20 bis 100°C, vorzugsweise zwischen 60 bis 80°C.

Das Molverhältnis 4-Halogen-3-($C_1$—$C_2$)-alkoxy-2-E-Butensäure-($C_1$—$C_4$)-alkylester zu $NH_3$ liegt auch hier zweckmässig zwischen 1 zu 2 und 1 zu 5, vorteilhaft zwischen 1 zu 2,5 und 1 zu 3,5.

Nach üblicher Aufarbeitung, z.B. durch Extraktion mit geeigneten Lösungsmitteln aus der Gruppe der halogenierten Kohlenwasserstoffe, wie u.a. Methylenchlorid und Chloroform, kann das entsprechende 4-($C_1$—$C_2$)-alkoxya-3-pyrrolin-2-on in guter Ausbeute erhalten werden.

Dieses kann gegebenenfalls zusätzlich durch z.B. Umkristallisieren in aromatischen Kohlenwasserstoffen, bevorzugt in Toluol, gereinigt werden.

## Beispiel 1

a) *Herstellung von 4-Chlor-3-methoxy-2-E-butensäuremethylester*

31,0 g (0,2 Mol) 4-Chloracetessigsäuremethylester wurden mit 106,0 g (1,0 Mol) Orthoameisensäuretrimethylester vermischt. Unter Argon gab man 30,0 g Amberlyst-15-Ionenaustauscherharz unter Rühren hinzu. Unter heftiger Gasentwicklung stieg die Reaktionstemperatur auf 40°C. Nach 5 Stunden Rühren liess sich im Dünnschichtchromatogramm kein Edukt mehr nachweisen. Man filtrierte vom Ionen-

austauscherharz ab und destillierte den Rückstand im Wasserstrahlvakuum. Das Destillat wurde mit 1,0 g p-Toluolsulfonsäure-monohydrat versetzt und langsam auf 150°C erhitzt, wobei Methanol abdestillierte.

Die Reaktionsmasse wurde anschliessend im Wasserstrahlvakuum destilliert.

Man erhielt 24,7 g einer farblosen Flüssigkeit mit einem Siedepunkt $Kp_{12}$ = 93°C.

NMR ($CDCl_3$) δ = 5,16 (s, 1H); 4,67 (s, 2H); 3,73 (s, 6H).

Ausbeute: 75%.

b) *Herstellung von 4-Methoxy-3-pyrrolin-2-on*

32,9 g (0,2 Mol) 4-Chlor-3-methoxy-2-E-butensäuremethylester wurden mit 82,0 g (1,2 Mol) 25%-ig wässriger $NH_3$-Lösung versetzt und unter kräftigem Rühren auf 80°C erhitzt. Nach 1 Stunde Nachrühren liess man auf Raumtemperatur abkühlen und extrahierte die wässrige Lösung viermal mit je 200,0 ml Methylenchlorid. Nach Trocknen der organischen Phase über $Na_2SO_4$ wurde abfiltriert, eingedampft und der Rückstand aus 120,0 ml Toluol heiss umkristiallisiert.

Man erhielt 16,9 g weisses, kristallines Produkt vom Schmelzpunkt 132 bis 133°C.

Ausbeute: 74,8%

NMR ($CDCl_3$) δ = 6,92 (br. s, 1H); 5,07 (s, 1H), 3,93 (s, 2H); 3,82 (s, 3H).

c) *Herstellung von 4-Methoxy-3-pyrrolin-2-on-1-yl-essigsäureethylester*

11,3 g (0,1 Mol) 4-Methoxy-3-pyrrolin-2-on und 24,2 g (0,137 Mol) Bromessigsäureethylester (95%-ig) wurden in 150,0 ml Acetonitril suspendiert. Bei 0°C versetzte man die Suspension mit 4,1 g (0,137 Mol) 80%-igem Natriumhydrid. Anschliessend liess man die Reaktionslösung auf Raumtemperatur erwarmen und rührte 90 Minuten nach. Nach Abdampfen des Lösungsmittels wurde der Rückstand mit 150,0 ml Eiswasser aufgeschlämmt und viermal mit je 400,0 ml Methylenchlorid extrahiert. Nach üblicher Aufarbeitung wurde der obige Rückstand mit 100,0 ml Petrolether (50 bis 70°C) versetzt und über Nacht im Kühlschrank aufbewahrt. Das auskristallisierte Produkt wurde abgenutscht und im Hochvakuum getrocknet.

Man erhielt 19,6 g gelblich gefärbtes Produkt vom Schmelzpunkt 55 bis 58°C.

Ausbeute: 98,5%

NMR ($CDCl_3$) δ = 5,11 (s, 1H); 4,69 (q, J=7,0 Hz, 2H); 4,66 (s, 2H); 3,99 (3, 2H); 3,83 (s, 3H); 1,28 (t, J=7,0 Hz, 3H);

MS (70 eV) m/z = 199 ($M^+$, 25); 126 (100).

Beispiel 2

a) *Herstellung von 4-Methoxy-3-pyrrolin-2-on*

Eine Lösung von 63,3 g (0,409 Mol) 4-Chloracetessigsäuremethylester und 54,0 g (0,508 Mol) Orthoameisensäuremethylester wurde auf 10°C gekühlt und innerhalb von 10 Minuten mit 2,0 g (0,02 Mol) konz. $H_2SO_4$ versetzt. Man liess bei Raumtemperatur 5 Stunden nachrühren. Vom intermediär entstehenden 4-Chlor-3,3-di-methoxy-butansäuremethylester wurden anschließend die flüchtigen Produkte bei einem Vakuum von 25 mbar abdestilliert. Dann wurde der Rückstand bei 125°C und 100 mbar 2,5 Stunden erhitzt. Während dieser Zeit distillierten 10,5 g Methanol ab. Der Rückstand wurde innerhalb von 1,75 Stunden tropfenweise bei 64 bis 68°C zu einer Lösung von 121,3 g (17%-ig) konz. $NH_3$-Lösung in 83,7 g Eiswasser gegeben. Nach der Hälfte der Zugabe gab man 24,3 g (17%-ig) konz. $NH_3$-Lösung hinzu. Nach beendeter Zugabe liess man die Reaktionslösung bei 65°C noch 45 Minuten nachrühren. Anschliessend kühlte man auf Raumtemperatur ab und extrahierte die Reaktionslösung kontinuierlich (12 Stunden) mit 300 ml Methylenchlorid. Die Methylenchlorid-Lösung wurde über $Na_2SO_4$ getrocknet und abfiltriert. Nach Eindampfen der Lösung unter Vakuum wurde der kristalline Rückstand aus 150 ml Toluol heiss umkristallisiert.

Man erhielt 40,8 g weisses, kristallines Produkt mit einem Schmelzpunkt von 130 bis 132°C (Gehalt (GC): 99,0%).

Dies entsprach einer Ausbeute von 87,4%.

b) *Herstellung von 4-Methoxy-3-pyrrolin-2-on-1-yl-essigsäureethylester*

4,27 g 4-Methoxy-3-pyrrolin-2-on und 8,30 g Bromessigsäureethylester wurden in 50 ml Dimethylformamid gelöst und bei 0°C innerhalb von 20 Minuten in 3 Portionen unter kräftigem Rühren und Argon mit 1,41 g Natriumhydrid versetzt.

Nach 90 Minuten Nachreaktionszeit bei 0°C wurde mit verdünnter Salzsäure neutralisiert und das Lösungsmittel im Vakuum abgedampft. Der Rückstand wurde in 50 ml Eiswasser aufgenommen und zweimal mit je 50 ml Methylenchlorid extrahiert. Die organische Phase wurde über $Na_2SO_4$ getrocknet und am Rotationsverdampfer unter Vakuum eingedampft. Der Rückstand kristallisierte bei Raumtemperatur langsam durch.

Man erhielt 8,0 g Produkt mit einem Schmelzpunkt von 54 bis 56°C und einem Gehalt nach GC von 90,8%.

Dies entsprach 7,26 g 100%-igem Produkt ≙ 97,4% Ausbeute.

Beispiel 3

*Herstellung von 4-Methoxy-3-pyrrolin-2-on-1-yl-essigsäuremethylester*

Analog erhielt man aus 4-Methoxy-3-pyrrolin-2-on und Bromessigsäuremethylester in Dimethylformamid mit Natriumhydrid bei 0°C den 4-methoxy-3-pyrrolin-2-on-1-yl-essigsäuremethylester in 92% Ausbeute mit einem Schmelzpunkt von 104 bis 106°C.

Beispiel 4

*Herstellung von 4-Methoxy-3-pyrrolin-2-on-1-yl-essigsäurebenzylester*

Analog erhielt man mit Bromessigsäurebenzylester in Acetonitril mit Natriumhybrid bei 0°C den 4-Methoxy-3-pyrrolin-2-on-1-yl-essigsäurebenzylester in 95% Ausbeute mit einem Schmelzpunkt von 129 bis 130°C.

Beispiel 5

*4-Methoxy-3-pyrrolin-2-on-1-yl-essigsäuremethylester aus 4-Chlor-3-methoxy-buten-2-E-säuremethylester*

Eine Suspension von 6,5 g (0,052 Mol) Glycinmethylester-hydrochlorid in 30 ml Acetonitril wurde mit einer Lösung von 5,3 g Triethylamin in 10 ml Acetonitril versetzt. Man erhitzte zum Rückfluss und gab 2,1 g wasserfreies Natriumacetat hinzu. Anschliessend wurde innerhalb von 30 Minuten ein Lösung von 4,4 g (0,025 Mol) 4-Chlor-3-methoxy-buten-2-E-säuremethylester (Gehalt (GC): 95,2%) und 20 ml Acetonitril zugetropft. Man liess unter Rückfluss 4,5 Stunden nachrühren. Dann wurde die Reaktionslösung auf 0°C abgekühlt, vom ausgefallenen Feststoff abfiltriert und das Filtrat am Rotationsverdampfer eingeengt. Der Rückstand wurde in 100 ml Eiswasser aufgenommen, mit 6,0 g 32%-iger Salzsäure angesäuert und fünfmal mit je 100 ml Methylenchlorid extrahiert. Die organische Lösung wurde über $Na_2SO_4$ getrocknet und eingedampft. Der Rückstand wurde in 30 ml Tetrachlorkohlenstoff umkristallisiert. Man erhielt 4,0 g weisses, kristallines Produkt vom Schmelzpunkt: 150 bis 107°C (GC-Gehalt: 96%). Ausbeute: 81,5%.

**Patentansprüche**

1. 4-Alkoxy-3-pyrrolin-2-on-1-yl-essigsäure-$C_1$—$C_4$-alkyl- oder -benzylester der Formel

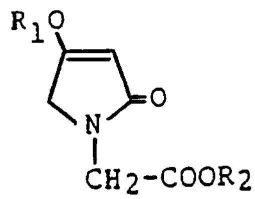

(1)

worin $R_1$ $C_1$—$C_2$-Alkyl und $R_2$ $C_1$—$C_4$-Alkyl oder Benzyl bedeuten.

2. Verbindung nach Patentanspruch 1, nämlich 4-Methoxy-3-pyrrolin-2-on-1-yl-essigsäureethylester der Formel

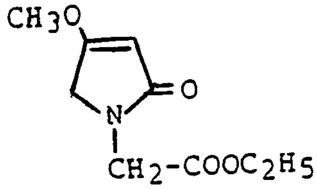

(2)

3. Verfahren zur Herstellung von Verbindungen gemäß Patentanspruch 1, dadurch gekennzeichnet, daß man in einem ersten Schritt einen 4-Halogenacetessigsäure-$C_1$—$C_4$-alkylester mit einem Orthoameisensäure-$C_1$—$C_2$-trialkylester in saurem Medium umsetzt, den entstehenden 4-Halogen-3-($C_1$—$C_2$)-alkoxy-2-E-butensäure-$C_1$—$C_4$-alkylester gegebenenfalls isoliert, dann in einem zweiten Schritt

a) entweder direkt mit einem Glycin-$C_1$—$C_4$-alkyl- oder -benzylester in Gegenwart einer Base zum Endprodukt umsetzt oder

b) zuerst mit wäßriger Ammoniaklösung zum 4-($C_1$—$C_2$)-Alkoxy-3-pyrrolin-2-on umsetzt, wonach man mit 2-Bromessigsäure-$C_1$—$C_4$-alkyl- oder -benzylester in Gegenwart eines Alkalihydrids zum Endprodukt gelangt.

4. Verfahren gemäß Patentanspruch 3, dadurch gekennzeichnet, daß man zur Erzeugung des sauren Mediums Schwefelsäure, Suflonsäuren oder einen sauren Ionenaustauscher anwendet.

5. Verfahren gemäß mindestens einem der Patentansprüche 3—4, dadurch gekennzeichnet, daß man als Base für die Umsetzung der Stufe a) mit dem Glycinester schwache anorganische Basen oder tertiäre aliphatische Amine verwendet.

6. Verfahren gemäß mindestens einem der Patentansprüche 3—5, dadurch gekennzeichnet, daß man als Lösungsmittel für die Umsetzung der Stufe a) mit Glycinester aprotische oder protische Lösungsmittel mit hoher Polarität verwendet.

7. Verfahren gemäß mindestens einem der Patentansprüche 3—6, dadurch gekennzeichnet, daß man für die Umsetzung der Stufe a) mit dem Glycinester eine Reaktionstemperatur von 60 bis 120°C wählt.

8. Verfahren gemäß mindestens einem der Patentansprüche 3—7, dadurch gekennzeichnet, daß man für die Umsetzung der Stufe b) zum 4-($C_1$—$C_2$)-Alkoxy-3-pyrrolin-2-on-1-yl-essigsäure-($C_1$—$C_4$)-alkylester ein Molverhältnis von 4-($C_1$—$C_2$)-Alkoxy-3-pyrrolin-2-on zu Bromessigsäure-$C_1$—$C_4$-alkylester oder Bromessigsäurebenzylester und zu Alkalihydrid zwischen 1 zu 1 zu 1 und 1 zu 1,5 zu 1,4 wählt.

9. Verfahren gemäß mindestens einem der Patentansprüche 3—8, dadurch gekennzeichnet, daß man für die Umsetzung der Stufe b) zum Endprodukt eine Reaktionstemperatur zwischen 0 und 50°C einstellt.

10. Verfahren gemäß Patentanspruch 3, dadurch gekennzeichnet, daß man im ersten Schritt den durch Umsetzung von 4-Halogenacetessigsäure-$C_1$—$C_4$-alkylester und Orthoameisensäure-$C_1$—$C_2$-trialkylester in saurem Medium zunächst entstehenden 4-Halogen-3,3-di-[($C_1$—$C_2$)-alkoxy]butansäure-$C_1$—$C_4$-alkylester durch Erhitzen im Vakuum in den 4-Halogen-3-($C_1$—$C_2$)-alkoxy-2-E-butensäure-$C_1$—$C_4$-alkylester überführt, gegebenenfalls isoliert und dann im zweiten Schritt gemäß b) von Patentanspruch 3 verfährt.

11. Verfahren gemäß Patentanspruch 10, dadurch gekennzeichnet, daß zur Erzeugung des sauren Mediums Schwefelsäure oder Sulfonsäuren verwendet werden.

12. Verfahren gemäß mindestens einem der Patentansprüche 10—11, dadurch gekennzeichnet, daß die Überführung in den 4-Halogen-3-($C_1$—$C_2$)-alkoxy-2-E-butensäure-$C_1$—$C_4$-alkylester bei einem Druck von 50 bis 200 mbar und einer Temperatur von 100 bis 160°C erfolgt.

13. Verfahren gemäß mindestens einem der Patentansprüche 10—12, dadurch gekennzeichnet, daß man für die Umsetzung zum 4-($C_1$—$C_2$)-Alkoxy-3-pyrrolin-2-on wäßrige $NH_3$-Lösung einer Konzentration zwischen 10% und 25% anwendet.

14. Verfahren gemäß mindestens einem der Patentansprüche 10—13, dadurch gekennzeichnet, daß man für die Umsetzung zum 4-($C_1$—$C_4$)-Alkoxy-3-pyrrolin-2-on das Molverhältnis 4-Halogen-3-($C_1$—$C_2$)-alkoxy-2-E-butensäure-$C_1$—$C_4$-alkylester zu $NH_3$ zwischen 1 zu 2 und 1 zu 5 wählt.

15. Verfahren gemäß mindestens einem der Patentansprüche 10—14, dadurch gekennzeichnet, daß man die Umsetzung zum 4-($C_1$—$C_2$)-Alkoxy-3-pyrrolin-2-on bei Temperaturen zwischen 20 und 100°C ausführt.

**Revendications**

1. Esters alcoyliques en $C_1$—$C_4$ ou esters benzyliques d'acides 4-alcoxy-3-pyrroline-2-one-1-yl-acétiques de formule

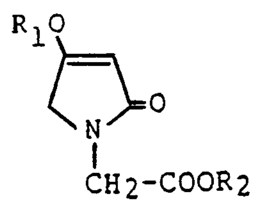

(1)

dans laquelle $R_1$ représente alcoyle en $C_1$—$C_2$ et $R_2$ représente alcoyle en $C_1$—$C_4$ ou benzyle.

2. Composé selon la revendication 1, à savoir le 4-méthoxy-3-pyrroline-2-one-1-yl-acétate d'éthyle de formule

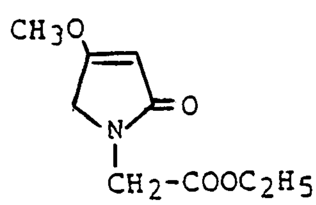

(2)

3. Procédé pour la préparation des composés selon la revendication 1, caractérisé en ce que dans une première étape on fait réagir un ester alcoylique en $C_1$—$C_4$ d'un acide 4-halogénoacétylacétique avec un ester trialcoylique en $C_1$—$C_2$ de l'acide orthoformique en milieu acide, on isole éventuellement l'ester alcoylique en $C_1$—$C_4$ d'acide 4-halogéno-3-alcoxy(en $C_1$—$C_2$)-2-E-butènoïque résultant, puis dans une deuxième étape.

a) soit on fait réagir directement avec un ester alcoylique en $C_1$—$C_4$ ou un ester benzylique de la glycine en présence d'une base pour donner le produit final

b) soit on fait réagir d'abord avec une solution aqueuse d'ammoniaque pour donner la 4-alcoxy(en

$C_1$—$C_2$)-3-pyrroline-2-one, à la suite de quoi on parvient au produit final avec de l'ester alcoylique en $C_1$—$C_4$ ou benzylique de l'acide 2-bromoacétique en présence d'un hydrure alcalin.

4. Procédé selon la revendication 3, caractérisé en ce que pour produire le milieu acide, on a recours à de l'acide sulfurique, à des acides sulfoniques ou à un échangeur d'ions acide.

5. Procédé selon au moins l'une des revendications 3—4, caractérisé en ce que l'on utilise comme bases pour la réaction de l'étape a) avec l'ester de glycine, des bases non organiques faibles ou des amines aliphatiques tertiaires.

6. Procédé selon au moins l'une des revendications 3—5, caractérisé en ce que l'on utilise comme solvants pour la réaction de l'étape a) avec l'ester de glycine, des solvants aprotiques ou protiques ayant une polarité élevée.

7. Procédé selon au moins l'une des revendications 3—6, caractérisé en ce que l'on choisit, pour la réaction de l'étape a) avec l'ester de glycine, une température de réaction de 60 à 120°C.

8. Procédé selon au moins l'une des revendications 3—7, caractérisé en ce que, pour la réaction de l'étape b) conduisant à l'ester alcoylique en $C_1$—$C_4$ de l'acide 4-alcoxy(en $C_1$—$C_2$)-3-pyrroline-2-one-1-yl-acétique, on choisit un rapport molaire de la 4-alcoxy(en $C_1$—$C_2$)-3-pyrroline-2-one au bromoacétate d'alcoyle en $C_1$—$C_4$ ou au bromoacétate de benzyle et à l'hydrure d'alcalin compris entre 1 à 1 à 1 et 1 à 1,5 à 1,4.

9. Procédé selon au moins l'une des revendications 3—8, caractérisé en ce que, pour la réaction de l'étape b) pour donner le produit final, on établit une température de réaction entre 0 et 50°C.

10. Procédé selon la revendication 3, caractérisé en ce que dans la première étape, le 4-halogéno-3,3-di-[alcoxy(en $C_1$—$C_2$)]butanoate d'alcoyle en $C_1$—$C_4$ résultant d'abord en milieu acide de la réaction du 4-halogénoacétylacétate d'alcoyle en $C_1$—$C_4$ et de l'ester trialcoylique en $C_1$—$C_2$ de l'acide orthoformique, est transformé par chauffage sous vide en 4-halogéno-3-alcoxy(en $C_1$—$C_2$)-2-E-butènoate d'alcoyle en $C_1$—$C_4$, éventuellement isolé puis traité dans la deuxième étape selon b) de la revendication 3.

11. Procédé selon la revendication 10, caractérisé en ce que pour produire le milieu acide, on utilise de l'acide sulfurique ou des acides sulfoniques.

12. Procédé selon au moins l'une des revendications 10—11, caractérisé en ce que la transformation en 4-halogéno-3-alcoxy(en $C_1$—$C_2$)-2-E-butènoate d'alcoyle en $C_1$—$C_4$ a lieu sous une pression de 50 à 200 mbar et à une température de 100 à 160°C.

13. Procédé selon au moins l'une des revendications 10—12, caractérisé en ce que pour la transformation en 4-alcoxy(en $C_1$—$C_2$)-3-pyrroline-2-one, on utilise une solution aqueuse de $NH_3$ à une concentration comprise entre 10% et 25%.

14. Procédé selon au moins l'une des revendications 10—13, caractérisé en ce que pour la transformation en 4-alcoxy(en $C_1$—$C_4$)-3-pyrroline-2-one, le rapport molaire du 4-halogéno-3-alcoxy(en $C_1$—$C_2$)-2-E-butènoate d'alcoyle en $C_1$—$C_4$ au $NH_3$ est choisi entre 1 à 2 et 1 à 5.

15. Procédé selon au moins l'une des revendications 10—14, caractérisé en ce qu'on effectue la transformation en 4-alcoxy(en $C_1$—$C_2$)-3-pyrroline-2-one à des températures entre 20 et 100°C.

**Claims**

1. 4-Alkoxy-3-pyrrolin-2-one-1-yl-acetic acid-$C_1$—$C_4$-alkyl or benzyl esters of the formula

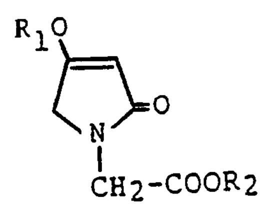

(1)

wherein $R_1$ is $C_1$—$C_2$-alkyl and $R_2$ is $C_1$—$C_4$-alkyl or benzyl.

2. Compound according to patent claim 1, namely the 4-methoxy-3-pyrrolin-2-one-1-yl-acetic acid ethyl ester of the formula

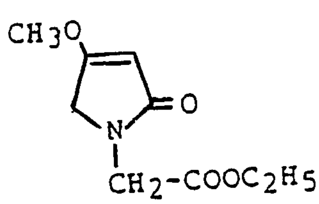

(2)

3. Process for the preparation of compounds according to patent claim 1, characterized in that in a first step a 4-halo-aceto-acetic acid-$C_1$—$C_4$-alkyl ester is reacted with an orthoformic acid-$C_1$—$C_2$-trialkyl ester in

an acidic medium, the resulting 4-halo-3-($C_1$—$C_2$)-alkoxy-2-E-butenoic acid-$C_1$—$C_4$-alkyl ester is, if desired, isolated, whereupon in a second step

a) it is reacted either directly in the presence of a base with a glycine-$C_1$—$C_4$-alkyl or benzyl ester to the end product or

b) it is first reacted with aqueous ammonia solution to 4-($C_1$—$C_2$)-alkoxy-3-pyrrolin-2-one whereupon the end product is obtained with 2-bromo-acetic acid—$C_1$—$C_4$-alkyl or benzyl esters in the presence of an alkali hydride.

4. Process according to patent claim 3, characterized in that for the formation of the acidic medium sulfuric acid, sulfonic acids or an acidic ion exchanger is used.

5. Process according to at least one of patent claims 3 to 4, characterized in that as base for the reaction of step a) with the glycine ester weak inorganic bases or tertiary aliphatic amines are used.

6. Process according to at least one of patent claims 3 to 5, characterized in that as solvent for the reaction of step a) with the glycine ester aprotic or protic solvents having a high poilarity are used.

7. Process according to at least one of patent claims 3 to 6, characterized in that for the reaction of step a) with the glycine ester a reaction temperature of 60 to 120°C is chosen.

8. Process according to at least one of patent claims 3 to 7, characterized in that for the reaction of step b) into the 4-($C_1$—$C_2$)-alkoxy-3-pyrrolin-2-one-1-yl-acetic acid-($C_1$—$C_4$)-alkyl ester a molar ratio of 4-($C_1$—$C_2$)-alkoxy-3-pyrrolin-2-one to bromo-acetic acid-$C_1$—$C_4$-alkyl ester or bromoacetic acid benzyl ester and to alkali hydride between 1:1:1 and 1:1.5:1.4 is chosen.

9. Process according to at least one of patent claims 3 to 8, characterized in that for the reaction of step b) to the end product a reaction temperature between 0 and 50°C is used.

10. Process according to patent claim 3, characterized in that in a first step the 4-halo-3,3-di-[($C_1$—$C_2$)-alkoxy]-butanoic acid-$C_1$—$C_4$-alkyl ester, obtained by reaction of 4-halo-acetoacetic acid-$C_1$—$C_4$-alkyl ester and orthoformic acid-$C_1$—$C_2$-trialkyl ester in an acidic medium, is transformed by heating in vacuo into the 4-halo-3-($C_1$—$C_2$)-alkoxy-2-E-butenoic acid-$C_1$—$C_4$-alkyl ester, if desired, isolated, whereupon the second step according to b) of patent claim 3 is carried out.

11. Process according to patent claims 10, characterized in that for the formation of the acidic medium sulfuric acid or sulfonic acids are used.

12. Process according to at least one of patent claims 10 to 11, characterized in that the transformation into the 4-halo-3-($C_1$—$C_2$)-alkoxy-2-E-butenoic acid-$C_1$—$C_4$-alkyl ester is effected at a pressure of 50 to 200 mbar and a temperature of 100 to 160°C.

13. Process according at least one of patent claims 10 to 12, characterized in that for the reaction into 4-($C_1$—$C_4$)-alkoxy-3-pyrrolin-2-one an aqueous $NH_3$-solution having a concentration between 10 and 25% is used.

14. Process according to at least one of patent claims 10 to 13, characterized in that for the reaction into 4-($C_1$—$C_4$)-alkoxy-3-pyrrolin-2-one the molar ratio of 4-halo-3-($C_1$—$C_2$)-alkoxy-2-E-butenoic acid-$C_1$—$C_4$-alkyl ester to $NH_3$ is chosen between 1:2 and 1:5.

15. Process according to at least one of patent claims 10 to 14, characterized in that the reaction into 4-($C_1$—$C_2$)-alkoxy-3-pyrrolin-2-one is carried out at temperatures between 20 and 100°C.